# EUROPEAN PATENT SPECIFICATION

(11) **EP 1 613 383 B1**
(45) Date of publication and mention of the grant of the patent: **29.10.2008**
(21) Application number: 04724220.1
(22) Date of filing: 29.03.2004
(51) Int. Cl.: A61M 16/01

(54) **SYSTEM, COMPONENT AND METHOD FOR DISTRIBUTION OF ANAESTHETICS WITH LOW BOILING POINT, WHERE A PRESSURE CONTROLLED VALVE OPENS THE ANAESTHETIC LIQUID CONTAINER**
SYSTEM, KOMPONENTE UND VERFAHREN ZUM VERTEILEN VON ANÄSTHETIKUM MIT NIEDRIGEM SIEDEPUNKT, WOBEI EIN DRUCKKONTROLLIERTES VENTIL DEN BEHÄLTER MIT DER NARKOSEFLÜSSIGKEIT ÖFFNET
SYSTEME, COMPOSANT ET PROCEDE DE DISTRIBUTION DE PRODUITS ANESTHESIANTS A POINT BAS D'EBULLITION, VALVE A PRESSION CONTROLEE OUVRANT LE CONTENANT DE LIQUIDE ANESTHESIANT

(30) Priority: 31.03.2003 SE 0300918
(43) Date of publication of application: 11.01.2006
(73) Proprietor: Sedana Medical AB, 174 57 Sundbyberg (SE)
(72) Inventor: LAMBERT, Hans, S-113 51 Stockholm (SE)
(74) Representative: Karlsson, Leif Karl Gunnar
(86) International application number: PCT/SE2004/000480
(87) International publication number: WO 2004/087244

(56) References cited:
- DE-C1- 10 106 010
- US-A- 4 750 483
- US-A- 5 197 462
- US-A- 5 235 971
- US-A- 5 243 973
- US-A- 5 335 652
- US-A- 6 155 255

## Description

### Field of the Invention

The present invention relates in a first aspect to a system for anaesthetic agent distribution, which system comprises an anaesthetic agent container and an anaesthetic gas pipe.

In additional aspects, the invention relates to a method for conversion of anaesthetic agent in liquid phase to anaesthetic agent in gas phase.

### Background of the Invention

In anaesthetic treatment of a patient, a usually occurring method is to supply the anaesthetic agent to the patient with the inhalation air, the anaesthetic agent on that occasion being in gas phase. The anaesthetic agent is normally provided in liquid state. This means that the agent has to be evaporated before it can be supplied to the patient. Vaporizers for this purpose are disclosed, among other things, in WO97/14465, WO97/36628, WO98/20926 and WO98/44977. US-A-5243973 discloses a system accordin g to the preamble of claim 1. The float valve 17 controls the outlet of vaporized anesthetic into the gas line 18.

It is important that the supply of the anaesthetic agent to the patient takes place evenly and at a controlled quantity. Anaesthetic liquid normally has a relatively low boiling point. This means that there is a risk that the anaesthetic liquid under certain conditions will transform into gas phase already in the liquid container from which the agent via a pipe is supplied to the vaporizer or that this takes place in said pipe. This creates gas pockets in the anaesthetic liquid that leads to the vaporizer. The consequence will be that the supply becomes uneven and uncontrolled with the risk of the anaesthetic treatment of the patient being disrupted or even becoming erroneous.

There are different types of anaesthetic agents, which have different boiling points. For certain anaesthetic agents such as, e.g., isoflurane and sevoflurane, the boiling point is about 40-60 °C. For such anaesthetic agents, the described problem does not normally occur, since the ambient temperature rarely reaches this value. Certain anaesthetic agents have, however, substantially lower boiling points. For, e.g., desflurane, the boiling point is 23 °C. For anaesthetic agents having relatively low boiling points, the risk of unintentional evaporation taking place already before the vaporizer, becomes considerably accentuated.

Against this background, the object of the present invention is to eliminate or at least reduce the risk of unintentional evaporation of the anaesthetic agent before it has reached up to the anaesthetic gas pipe.

### Summary of the Invention

In the first aspect of the invention, the object set-up has been attained by the fact that a system of the kind defined in the preamble of claim 1 comprises the special features that at least one liquid valve is arranged to selectively connect the anaesthetic liquid container with the anaesthetic gas pipe, the liquid valve being arranged to assume a closed position when the pressure in said anaesthetic liquid is below a predetermined value and to assume an open position when said pressure is equal to or exceeds said value.

Thanks to the valve, anaesthetic liquid will not be able to be supplied to the anaesthetic gas pipe unless the predetermined value is attained. By adapting this pressure level in a suitable way, an increased pressure can be secured in the anaesthetic liquid, which increases the boiling point thereof. Thereby, it is prevented that it unintentionally transforms into gas phase.

Thus, the risk of the above-described complications will be reduced. By way of controlling the pressure in the anaesthetic liquid in this manner, it is attained not only that an increased ambient temperature is prevented from creating the described drawbacks. The invention also ensures that an exceptionally high or low position of the container from which the anaesthetic liquid is supplied does not have a disturbing effect on the course of events, which otherwise could be the case because of the alteration of the pressure that such a position entails.

Thus, by means of the invented device, a safer anaesthetic treatment for the patient is obtained and enables a freer choice of the anaesthetic agent that can be used.

According to a preferred embodiment of the invented system, the anaesthetic liquid container is connected to the anaesthetic gas pipe via an anaesthetic liquid pipe.

By providing a pipe between the anaesthetic liquid container and the anaesthetic gas pipe, a greater flexibility with the system is attained thanks to the fact that the container within certain limits can be placed arbitrarily in relation to the anaesthetic gas pipe.

According to an additional preferred embodiment, the system comprises a plurality of liquid valves arranged in series.

Formed in this way, the system allows a greater freedom to locate the pressure control on different places in the system, e.g., in close connection to the anaesthetic liquid container or in close connection to the anaesthetic gas pipe, depending on the conditions in question. Two or more valves may also contribute to increase the reliability of the system.

According to an additional preferred embodiment, a liquid valve is arranged where the anaesthetic liquid pipe is connected to the anaesthetic gas pipe.

Locating the liquid valve on that place entails that the pressure control that the same exerts will control the pressure in the anaesthetic liquid in the entire system. Thereby, it will overcome problems of increased temperature not only in the liquid in the anaesthetic liquid container but also in the pipe that connects the same with the anaesthetic gas pipe.

According to an additional embodiment, a liquid valve is arranged at the connection of the anaesthetic liquid container to the anaesthetic liquid pipe. This position may in certain cases be the most practical one. Furthermore, by providing an anaesthetic liquid container with a liquid valve in the outlet, it means that the invention can be exercised also when the rest of the system is constructed from standard components of the conventional type.

According to an additional preferred embodiment, a liquid valve is arranged in the anaesthetic liquid pipe. This enables exercising of the invention also when the container and the connection to the anaesthetic gas pipe is of the conventional type.

According to an additional preferred embodiment, the anaesthetic liquid pipe is connected to the anaesthetic gas pipe via an evaporator formed as a porous body.

Such an evaporator is previously known, per se, and contributes to an efficient and even evaporation of the anaesthetic agent. The advantages of guaranteeing that the anaesthetic liquid is not evaporated prematurely become especially accentuated in connection with this type of vaporizer, since an even flow of anaesthetic liquid is important for the operation of such a one.

According to an additional preferred embodiment, a reflector is arranged in the anaesthetic gas pipe, which reflector is arranged to be able to absorb and desorb anaesthetic agents. Such a reflector is previously known, per se, and contributes to reducing the anaesthetic agent consumption by a part of the anaesthetic agent being re-used. Therefore, the new addition supplied from the anaesthetic liquid container has in each moment to be adapted to the quantity of anaesthetic agent that is re-circulated by means of the reflector. Therefore, here it is extra important with an even and controlled supply of anaesthetic liquid, and therefore the invention is especially advantageous to use when the system also comprises a reflector.

According to an additional preferred embodiment, the liquid valve is spring-operated. Thereby, the stated valve function may be provided automatically, the pressure in the anaesthetic liquid at the predetermined value overcoming the spring force so that the valve is opened. This eliminates the need for complicated external regulating equipment for the opening and closing of the valve.

According to an additional preferred embodiment, the liquid valve comprises flow-limiting means. In this way, it can be secured that the supplied amount of anaesthetic liquid is kept at a level corresponding to the need. The risk of uncontrolled overdosage is thus reduced.

According to an additional preferred embodiment, the spring of the liquid valve has adjustable spring force. Thereby, the predetermined value at which the valve is opened can be regulated and adjusted depending on the treatment situation in question and adapted to anaesthetic liquids of different boiling points.

According to an additional preferred embodiment, a motor driven pump is arranged to pump anaesthetic liquid from the container to the anaesthetic gas pipe. Thereby, an optimally even and controlled flow is provided, which is of particular importance in a system according to the invention.

The above-stated preferred embodiments of the invented system are defined in the claims depending on claim 1.

In a second aspect of the invention, the object set-up has been attained by a method of the kind defined in the preamble of claim 12 comprising the special measures that the anaesthetic liquid is brought into contact with a liquid valve, that the liquid valve is kept closed when the pressure in the anaesthetic liquid is below a predetermined value and that it is kept open when the pressure in the anaesthetic liquid is equal to or exceeds said value.

According to preferred embodiments of the invented method, the same is exercised by means of a system according to the invention.

Also by means of the invented method and preferred embodiments of the same, advantages of the type corresponding to what has been stated above for the invented system are gained.

According to a example of the use, the anaesthetic liquid is desflurane. This is one of the most important anaesthetic agents in this group, which makes the use of such an anaesthetic agent particularly important.

The invention is explained more closely in the subsequent detailed description of advantageous embodiment examples of the same, reference being made to the appended drawing figures.

### Brief Description of the Figures

Fig. 1 is a schematic illustration of a system of the type that the invention relates to.
Fig. 2 is an illustration of a first embodiment example of a system according to the invention.
Figs. 3 and 4 are corresponding illustrations of additional embodiment examples of a system according to the invention.
Fig. 5 is a schematical longitudinal section of an example of a liquid valve in the system according to the invention.
Figs. 6-8 are corresponding sections through alternative embodiment examples of liquid valves.
Fig. 9 is a schematic illustration of an example of a connection unit.
Fig. 10 is a corresponding illustration of an alternative example of a connection unit.
Fig. 11 is a schematic illustration of an example of an anaesthetic liquid container.
Fig. 12 is a schematic illustration of an example of an anaesthetic liquid pipe.

### Description of Advantageous Embodiment Examples

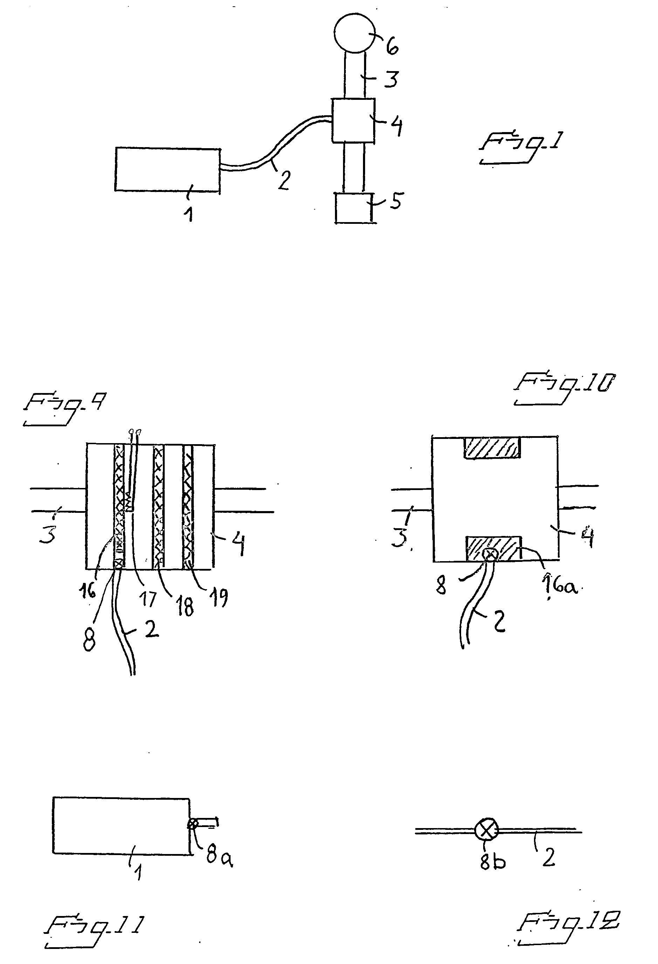

In fig. 1, a system of the type to which the invention relates is illustrated schematically. The system consists of an anaesthetic liquid container 1, which via an anaesthetic liquid pipe and a connection unit 4 supplies anaesthetic liquid to an anaesthetic gas pipe 3. The anaesthetic gas pipe 3 is at one end thereof connected to the respiratory organ of a patient 6 and at other end thereof to a respirator 5. The anaesthetic liquid is evaporated in the connection unit 4 and follows the inhalation air to the patient 6.

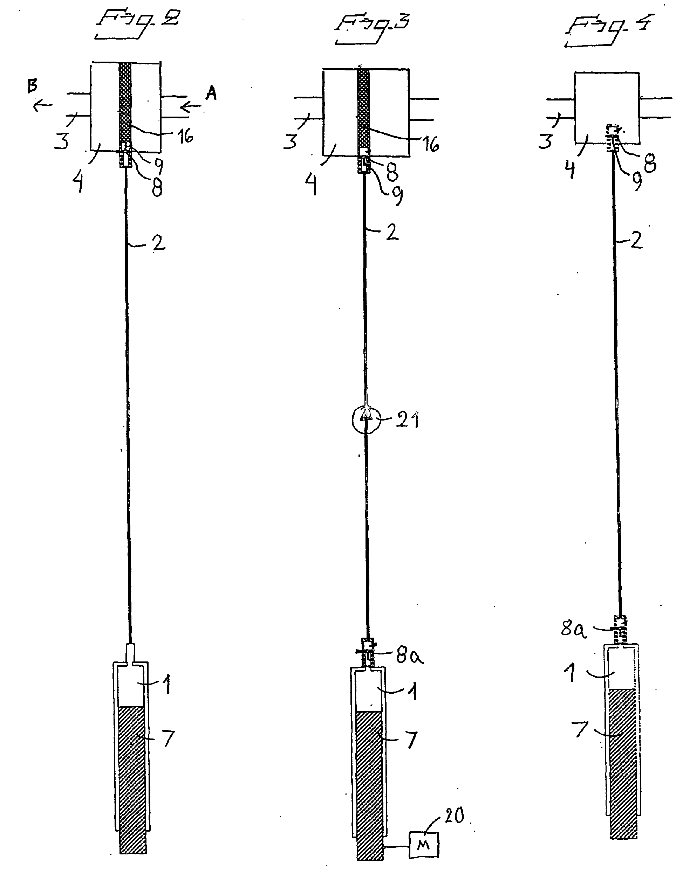

In fig. 2, a first example is shown of how such a system according to the invention may be formed. Here, the anaesthetic liquid container 1 consists of a syringe 1 with a piston 7 pushing out the anaesthetic liquid in the anaesthetic liquid pipe 2 to the connection unit 4. In the connection unit, a liquid valve 8 is arranged, which normally is kept closed by means of a spring 9. When the pressure in the anaesthetic liquid pipe 2 exceeds a predetermined value, e.g. 2 atm, the liquid pressure overcomes the spring force so that the liquid valve 8 opens. In that connection, anaesthetic liquid flows out to an evaporator 16. This is in the example formed as a bar and is of a porous material. Inhalation air from the respirator passes, as has been marked by the arrow A, the evaporator 16.

In that connection, the anaesthetic liquid will be exposed to the air flow over a large surface and evaporate easily. The evaporated anaesthetic agent will then follow the inhalation air to the patient, as has been marked by the arrow B. As has been described by way of introduction, the valve 8 has the function of maintaining a certain smallest pressure in the anaesthetic liquid in the syringe 1 and in the anaesthetic liquid pipe 2.

In fig. 3, a system is shown that differs from the one in fig. 2 by the fact that a liquid valve 8a is arranged also in the outlet of the syringe 1. The liquid valve 8a has the same function as the one 8 in the connection unit 4. Furthermore, the system in fig 3 is provided with a motor driven syringe pump 7 driven by a motor 20. In the pipe 2, a pump 21 is arranged as an alternative or as a complement. Also the embodiments according to figs 2 and 3 may naturally be provided with a motor driven 20 syringe pump 7 and/or a pump 21 in the pipe 2.

In fig. 4, an additional example is shown and that differs from the one in fig. 3 by the fact that the anaesthetic liquid is led directly out in the anaesthetic gas pipe without being dispersed in a porous body.

The examples in figs. 3 and 4 may further be modified so that the liquid valve 8 in the connection unit 4 is eliminated so that the pressure control is exerted only by the liquid valve 8a in the outlet of the syringe 1.

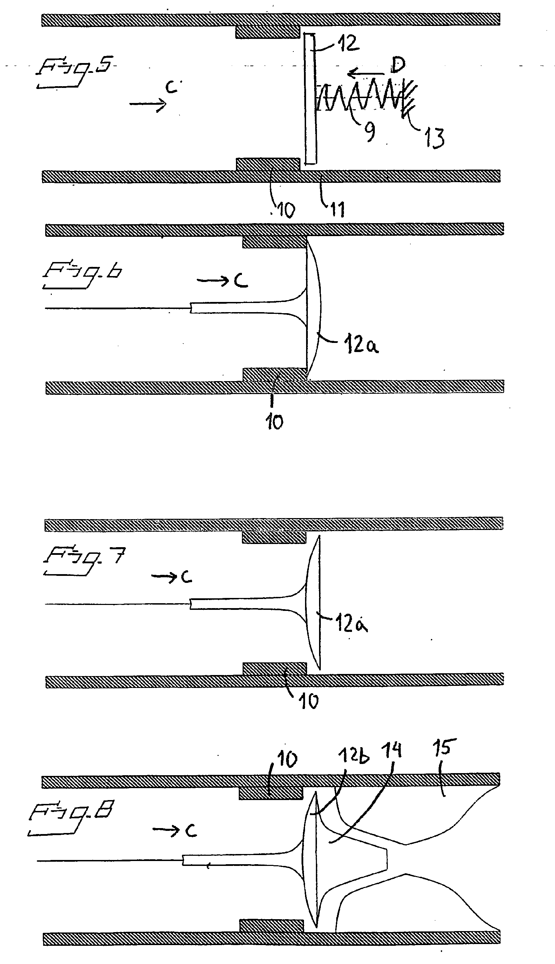

The liquid valves 8, 8a may be of an arbitrary type. In fig. 5, such a valve is illustrated in the simplest embodiment thereof. The direction of flow of the anaesthetic liquid is marked by the arrow C. A valve seat 10 is arranged in the circumferential direction next to the outer wall 11 of the valve. A valve body 12 is pressed to abutment against the valve seat 10 by the compression spring 9 by a force D. The figure shows the valve in the open position, i.e., when the pressure from the anaesthetic liquid overcomes the closing force from the spring 9. The spring 9 abuts against a fixed support 13. If it is desirable to make the force of the spring adjustable, this may be provided by means of a device that adjusts the axial position of the valve seat 10 or the support 13.

In fig. 6, an alternative example of the liquid valve is shown. Here, the valve body 12a is manufactured from a flexible material. When the pressure in the anaesthetic liquid on the upstream side of the valve, i.e., on the left side in the figure, exceeds a certain value, the liquid pressure will distort the elastic valve body 12a so that it assumes the position shown in fig. 7, in which the valve is open.

In fig. 8, an additional alternative example is shown. Here, the basic function is the same as for the valve in figs. 6 and 7. Furthermore, the valve in fig. 8 is provided with a flow-limiting device that consists of a projection 14 on the downstream side of the valve body 12b and a restriction formed by a ring-shaped body 15 located downstream the valve body 12b. The projection 14 and the body 15 have profiles adapted to each other in cross-section. The location formed therebetween limits the maximal flow rate through the valve.

In fig. 9, an alternative example of the connection unit 4 is shown. In addition to a porous body 16 as evaporator, additional components are arranged in the connection unit. Thus, a radiator 17 is arranged adjacent to the porous body 16 to which heat is brought, e.g. in electric way. The object is, by means of increased heat, to facilitate the evaporation. Furthermore, there is a reflector 19, i.e., a filter that absorbs anaesthetic agent from exhalation air and desorbs anaesthetic agent to inhalation air. Finally, there is a moisture filter 18 for water steam in order to absorb moisture from the exhalation air and desorb it to the inhalation air. All said components may be present in the connection unit or only some one of them. Reflector and moisture filter, respectively, may alternatively be arranged on another place in the anaesthetic gas pipe.

In fig. 10, an alternative example of evaporator is shown. Here, the same is formed as a ring-shaped body 16a.

In fig. 11, an example of anaesthetic liquid container 1 is shown provided with a liquid valve 8a.

In fig. 12, an example of anaesthetic liquid pipe 2 is shown, provided with a liquid valve 8b.

## Claims

1. System for anaesthetic agent distribution, which system comprises an anaesthetic liquid container and an anaesthetic gas pipe (3), **characterized in that** the system further includes at least one liquid valve (8, 8a, 8b) arranged to selectively connect the anaesthetic liquid container (1) with the anaesthetic gas pipe (3), the liquid valve (8, 8a, 8b) being arranged to assume a closed position when the pressure of said anaesthetic liquid is below a predetermined value and to assume an open position when said pressure is equal to or exceeds said value.

2. System according to claim 1, **characterized in that** the anaesthetic liquid container (1) is connected to the anaesthetic gas pipe (3) via an anaesthetic liquid pipe (2).

3. System according to claim 2, **characterized in that** the system comprises a plurality of liquid valves (8, 8a) arranged in series.

4. System according to claim 2 or 3, **characterized in that** a liquid valve (8) is arranged at the connection of the anaesthetic liquid pipe (2) to the anaesthetic gas pipe (3).

5. System according to any one of claims 2-4, **characterized in that** a liquid value (8a) is arranged at the connection of the anaesthetic liquid container to the anaesthetic liquid pipe (2).

6. System according to any one of claims 2-5, **characterized in that** a liquid valve (8b) is arranged in the anaesthetic liquid pipe (2).

7. System according to any one of claims 2-6, **characterized in that** the anaesthetic liquid pipe (2) is connected to the anaesthetic gas pipe (3) via an evaporator (9, 9a) formed as a porous body.

8. System according to any one of claims 1-7, **characterized in that** a reflector (19) is arranged in the anaesthetic gas pipe, which reflector (19) is arranged to be able to absorb and desorb anaesthetic agent.

9. System according to any one of claims 1-8, **characterized in that** the liquid valve (8) is spring-operated (9).

10. System according to claim 9, **characterized in that** the liquid valve comprises flow-limiting means (14, 15).

11. System according to claim 9 or 10, **characterized in that** the spring (9) of the liquid valve (8) has adjustable spring force.

12. System according to any one of claims 1-11, **characterized in that** the system comprises a motor driven pump (7, 20, 21) arranged to pump anaesthetic liquid from the anaesthetic agent container (1) to the anaesthetic gas pipe (3).

13. Method for conversion of anaesthetic agent in liquid phase to anaesthetic agent in gas phase, **characterized in that** the anaesthetic liquid is brought into contact with a liquid valve, that the liquid valve is kept closed when the pressure of the anaesthetic liquid is below a predetermined value and kept open when the pressure in the anaesthetic liquid is equal to or exceeds said value.

14. Method according to claim 13, **characterized in that** it is exercised by means of a system according to any one of claims 1-12.

## Patentansprüche

1. System zur Verteilung von Anästhesiemitteln, das einen Anästhesieflüssigkeitsbehälter und eine Anästhesiegasleitung (3) aufweist, **dadurch gekennzeichnet, dass** das System ferner wenigstens ein Flüssigkeitsventil (8, 8a, 8b) aufweist, das angeordnet ist, um den Anästhesieflüsthesieflüssigkeitsbehälter (1) mit der Anästhesiegasleitung (3) selektiv zu verbinden, wobei das Flüssigkeitsventil (8, 8a, 8b) dazu angeordnet ist, eine geschlossene Position einzunehmen, wenn der Druck der Anästhesieflüssigkeit unterhalb eines vorbestimmten Wertes ist, und eine offene Position einzunehmen, wenn der Druck gleich oder größer als der genannte Wert ist.

2. System nach Anspruch 1, **dadurch gekennzeichnet, dass** des Anästhesieflüssigkeitsbehälter (1) mit der Anästhesiegasleitung (3) über ein Anästhesieflüssigkeitsrohr (2) verbunden ist.

3. System nach Anspruch 2, **dadurch gekennzeichnet, dass** das System eine Mehrzahl von Flüssigkeitsventilen (8, 8a) aufweist, die in Folge angeordnet sind.

4. System nach Anspruch 2 oder 3, **dadurch gekennzeichnet, dass** ein Flüssigkeitsventil (8) an der Verbindung des Anästhesieflüssigkeitsrohrs (2) mit der Anästhesiegasleitung (3) angeordnet ist.

5. System nach irgendeinem der Ansprüche 2 bis 4, **dadurch gekennzeichnet, dass** ein Flüssigkeitsventil (8a) an der Verbindung des Anästhesieflüssigkeitsbehälters mit dem Anästhesieflüssigkeitsrohr (2) angeordnet ist.

6. System nach irgendeinem der Ansprüche 2 bis 5, **dadurch gekennzeichnet, dass** ein Flüssigkeitsventil (8b) in dem Anästhesieflüssigkeitsrohr (2) angeordnet ist.

7. System nach irgendeinem der Ansprüche 2 bis 6, **dadurch gekennzeichnet, dass** das Anästhesieflüssigkeitsrohr (2) mit der Anästhesiegasleitung (3) über einen Verdampfer (9, 9a) verbunden ist, der als poröse Körper ausgebildet ist.

8. System nach irgendeinem der Ansprüche 1 bis 7, **dadurch gekennzeichnet, dass** ein Reflektor (19) in dem Anästhesiegasrohr angeordnet ist, wobei der Reflektor (19) dazu ausgebildet ist, Anästhesiemittel zu absorbieren und zu desorbieren.

9. System nach irgendeinem der Ansprüche 1 bis 8, **dadurch gekennzeichnet, dass** das Flüssigkeitsventil (8) federbetrieben (9) ist.

10. System nach Anspruch 9, **dadurch gekennzeichnet, dass** das Flüssigkeitsventil ein Flussbegrenzungsmittel (14, 15) aufweist.

11. System nach Anspruch 9 oder 10, **dadurch gekennzeichnet, dass** die Feder (9) des Flüssigkeitsventils (8) eine einstellbare Federkraft hat.

12. System nach irgendeinem der Ansprüche 1 bis 11, **dadurch gekennzeichent, dass** das System eine motorgetriebene Pumpe (7, 20, 21) aufweist, die dazu angeordnet ist, Anästhesieflüssigkeit von dem Anasthesiemittelbehälter (1) zu dem Anästhesiegasrohr (3) zu pumpen.

13. Verfahren zum Konvertieren eines Anästhesiemittels aus der flüssigen Phase zu einem Anästhesiemittel in der Gasphase, **dadurch gekennzeichnet, dass** die Anästhesieflüssigkeit in Kontakt mit einem Flüssigkeitsventil gebracht wird, dass das Flüssigkeitsventil geschlossen bleibt, wenn der Druck in der Anästhesieflüssigkeit unterhalb eines vorbestimmten Wertes ist, und offen gehalten wirt, wenn der Druck in der Anästhesieflüssigkeit gleich oder größer dem genannten Wert ist.

14. Verfahren nach Anspruch 13, **dadurch gekennzeichnet, dass** es mit Hilfe eines Systems gemäß irgendeinem der Ansprüche 1 bis 12 durchgeführt wird.

## Revendications

1. Système pour la distribution d'un agent anesthésiant, lequel système comprend un récipient de liquide anesthésiant et un tuyau de gaz anesthésiant (3), **caractérisé en ce que** le système comprend en outre au moins une vanne de liquide (8, 8a, 8b) agencée pour raccorder sélectivement le récipient de liquide anesthésiant (1) avec le tuyau de gaz anesthésiant (3), la vanne de liquide (8, 8a, 8b) étant agencée pour prendre une position fermée lorsque la pression dudit liquide anesthésiant est inférieure à une valeur prédéterminée et pour prendre une position ouverte lorsque ladite pression est égale ou supérieure à ladite valeur.

2. Système selon la revendication 1, **caractérisé en ce que** le récipient de liquide anesthésiant (1) est raccordé au tuyau de gaz anesthésiant (3) via un tuyau de liquide anesthésiant (2).

3. Système selon la revendication 2, **caractérisé en ce que** le système comprend une pluralité de vannes de liquide (8, 8a) agencée en série.

4. Système selon la revendication 2 ou 3, **caractérisé en ce qu'**une vanne de liquide (8) est agencée au niveau du raccordement du tuyau de liquide anesthésiant (2) au tuyau de gaz anesthésiant (3).

5. Système selon l'une quelconque des revendications 2 à 4, **caractérisé en ce qu'**une vanne de liquide (8a) est agencée au niveau du raccordement du récipient de liquide anesthésiant au tuyau de liquide anesthésiant (2).

6. Système selon l'une quelconque des revendications 2 à 5, **caractérisé en ce qu'**une vanne de liquide (8b) est agencée dans le tuyau de liquide anesthésiant (2).

7. Système selon l'une quelconque des revendications 2 à 6, **caractérisé en ce que** le tuyau de liquide anesthésiant (2) est raccordé au tuyau de gaz anesthésiant (3) via un évaporateur (9, 9a) formé en tant que corps poreux.

8. Système selon l'une quelconque des revendications 1 à 7, **caractérisé en ce qu'**un réflecteur (19) est agencé dans le tuyau de gaz anesthésiant, lequel réflecteur (19) est agencé pour pouvoir absorber et désorber l'agent anesthésiant.

9. Système selon l'une quelconque des revendications 1 à 8, **caractérisé en ce que** la vanne de liquide (8) est actionnée par ressort (9).

10. Système selon la revendication 9, **caractérisé en ce que** la vanne de liquide comprend des moyens de limitation d'écoulement (14, 15).

11. Système selon la revendication 9 ou 10, **caractérisé en ce que** le ressort (9) de la vanne de liquide (8) a une force de rappel ajustable.

12. Système selon l'une quelconque des revendications 1 à 11, **caractérisé en ce que** le système comprend une pompe motorisée (7, 20, 21) agencée pour pomper le liquide anesthésiant du récipient (1) d'agent anesthésiant dans le tuyau de gaz anesthésiant (3).

13. Procédé pour convertir un agent anesthésiant en phase liquide en agent anesthésiant en phase gazeuse, **caractérisé en ce que** le liquide anesthésiant est amené en contact avec une vanne de liquide, de sorte que la vanne de liquide est maintenue fermée lorsque la pression du liquide anesthésiant est inférieure à une valeur prédéterminée et maintenue ouverte lorsque la pression du liquide anesthésiant est égale ou supérieure à ladite valeur.

14. Procédé selon la revendication 13, **caractérisé en ce qu'**il est réalisé au moyen d'un système selon l'une quelconque des revendications 1 à 12.
